# EUROPÄISCHE PATENTSCHRIFT

(11) **EP 1 059 984 B1**
(45) Veröffentlichungstag und Bekanntmachung des Hinweises auf die Patenterteilung: **05.06.2002**
(21) Anmeldenummer: 98905196.6
(22) Anmeldetag: 06.03.1998
(51) Int. Cl.: B01D 29/01, A61L 2/06

(54) **TRAY**
TRAY
PLATEAU

(43) Veröffentlichungstag der Anmeldung: 20.12.2000
(73) Patentinhaber: Oberdorfer, Harald, 9607 Mosnang (CH)
(72) Erfinder: OBERDORFER, Harald, CH-9607 Mosnang (CH); SUTER, Heinz, CH-8330 Pfäffikon (CH)
(74) Vertreter: Stocker, Kurt
(86) Internationale Anmeldenummer: CH9800093
(87) Internationale Veröffentlichungsnummer: WO9944717

(56) Entgegenhaltungen:
- DE-A- 19 627 044
- DE-C- 3 340 963
- US-A- 4 671 873
- US-A- 4 701 234
- US-A- 4 783 321

## Beschreibung

Die Erfindung bezieht sich auf ein Tray nach dem Oberbegriff des Anspruches 1.

Bei der Verwendung von medizinischen Arbeitsgeräten, die bei Untersuchungen oder Eingriffen am lebenden Körper mit Blut in Kontakt kommen könnten, müssen diese Geräte bis zur Verwendung steril sein. Nach der Verwendung werden sie gereinigt und/oder desinfiziert, sterilisiert und aufbewahrt. Der dabei entstehende Manipulieraufwand, sollte so klein wie möglich sein. Aus der DE 196 27 044 A1 ist ein Tray bekannt das sowohl als Waschkorb in einer Waschvorrichtung als auch als Sterilisations-Tray in eine Sterilisationsvorrichtung einschiebbar ist. Vor dem Sterilisieren wird das Tray oben und unten mit einem Filtereinschub verschlossen. Dazu werden Stücke aus einem dampfdurchlässigen Filterpapier, das Keime zurückhält, in Schlitze eingeführt und gegebenenfalls mit einer Klemmstelle darin festgehalten. Beim Transportieren der Instrumente nach Gebrauch zum Waschen, zur thermischen Desinfektion und zur Sterilisation müssen diese nicht mehr in die Hand genommen werden, sondern können im gleichen Tray verbleiben. Es hat sich nun gezeigt, dass das Einschieben der Filter umständlich ist. Zum Schutz des Filters vor Verletzungen durch mechanische Einwirkungen muss gegebenenfalls noch ein Abdeckelement aufgesetzt werden. Zudem ist trotz der eingeschobenen Filterpapiere keine Sterillagerung der Instrumente im Tray gewährleistet, weil ein lückenloser Abschluss um den Filter in den Tray-Innenraum nicht mit Sicherheit zu realisieren ist.

Es sind handelsübliche Trays bekannt die in einem zentralen Bodenbereich einen Lochbereich umfassen. Ausserhalb des Lochbereiches stehen an den Eckpunkten eines Rechteckes Gewindebolzen vom Boden in den Aufnahmebereich des Trays vor. Zwischen den Gewindebolzen wird ein Filterpapier eingelegt, das den Lochbereich abdeckt. Anschliessend wird ein Lochblech deckungsgleich mit der Lochung des Lochbereiches auf das Filterpapier gelegt. Die Gewindebolzen sind durch Bohrungen des Lochbleches geführt. Das Lochblech kann nun mit Muttern auf den Gewindebolzen gegen den Trayboden gepresst werden. Durch die dichte Klemmung des Filterpapiers kann ein Gas- oder Dampf-Austausch zwischen dem Tray-Innenraum und der Umgebung nur durch das Filterpapier erfolgen. Dadurch wird eine Sterillagerung im Tray möglich. Es hat sich aber gezeigt, dass der dazu nötige Material-, Herstellungs- und Manipulieraufwand aufgrund des zusätzlich eingesetzten Lochbleches und aufgrund dessen umständlicher Befestigungsweise sehr hoch ist. Zudem ist diese Lösung auf das Einlegen eines Filterpapieres von oben auf einen zumindest beim Einlegen unten liegenden Lochbereich eingeschränkt. Für einen Waschkorb gemäss der DE 196 27 044 A1 ist diese Lösung nicht geeignet, weil ja nach dem Waschen zuerst die Instrumente entnommen werden müssten, bevor das Filterpapier von oben eingelegt werden könnte.

Die erfindungsgemässe Aufgabe besteht nun darin ein Tray zu finden, bei dem ein ebener Lochbereich mit kleinem Aufwand durch flächiges Filtermaterial so abgeschlossen werden kann, dass der Gas- und Dampf-Austausch zwischen dem Tray-Innenraum und der Umgebung nur durch das Filtermaterial erfolgen kann. Es soll eine Sterillagerung im Tray über einen längeren Zeitraum gewährleistet werden.

Diese Aufgabe wird durch ein Tray mit den Merkmalen des Anspruches 1 gelöst. Die abhängigen Ansprüche beschreiben bevorzugte Ausführungsformen.

Um einen Filter zur Erzielung eines Sterilität garantierenden Abschlusses an einer Tray-Teilfläche mit Durchtrittsöffnungen anzuordnen, genügt es, dass eine geschlossene, um die Durchtrittsöffnungen verlaufende Dichtlinie ausgebildet ist, entlang der zu verbindende Trayteile mit zusammenwirkenden Pressflächen die Dichtung und Flachmterial festklemmbar machen. Durch die Elastizität der Dichtung wird ein genügend gutes Abschliessen auch mit kleinem Pressdruck und selbst bei nicht exakt planen Pressflächen gewährleistet. Auf ein aufwendiges Festschrauben einer Lochplatte kann verzichtet werden.

Die Verbindung der Dichtung mit dem Filterflachmaterial hat den zusätzlichen Vorteil, dass der Filter durch das Einpressen seiner Dichtung in eine entsprechende Nut auch an einer Unterseite angeordnet werden kann, ohne dass er sich von der Unterseite aufgrund der Schwerkraft löst. Nach dem Festklemmen der Dichtung zwischen den Pressflächen verhindert die Dichtung jedes Eindringen von Mikroorganismen um den Filter in den Tray-Innenraum. Aufgrund der Möglichkeit den Filter an einer Nut festzusetzen, kann etwa an der nach unten gerichteten Innenseite eines Deckelteils mit einem Lochbereich ein Filter angeordnet werden. Der Deckel mit dem Filter wird dann beispielsweise auf den geschlossenen Mantel eines Waschkorbes aufgesetzt, so dass die obere Stirnseite des Mantels gegen die Dichtung des Filters presst. Dadurch ist der Innenraum des Waschkorbes von oben nur durch den Filter zugänglich. Der Filter wird von der darüberliegenden Deckelfläche gegen Beschädigung geschützt. Analog zum Deckel kann an der unteren Stirnseite des Waschkorb-Mantels ein Boden mit eingelegtem Filter so befestigt werden, dass die Filterdichtung zwischen Boden und Mantel festgeklemmt ist. Zudem können Deckel und Boden als identische Abschlussteile ausgebildet werden und sind somit austauschbar. Um die gewünschte Dampfdurchdringung durch die eingesetzten Filter nicht unnötig zu behindern, sind die Lochanordnungen der Abschlussteile und des Waschkorbes deckungsgleich übereinanderliegend ausgebildet. Zum pressenden Festklemmen der Abschlussteile ist eine Klemmvorrichtung vorgesehen, die gegebenenfalls zwischen Deckel und Boden, vorzugsweise aber zwischen je einem Abschlussteil und dem Mantel kraftschlüssig wirkt.

Die Filter mit am flexiblen Filterflachmaterial befestigten Dichtungen können auch in Trays, die ausschliesslich für die Sterilisation und gegebenenfalls ein anschliessendes Sterillagern eingesetzt werden, vorteilhaft eingesetzt werden. Eine einfache Ausgestaltung eines solchen Sterilisationstrays umfasst etwa einen Boden-bzw. Deckelteil mit einem Lochbereich und einen mit einem Mantelbereich formschlüssig an diesen anlegbaren Deckel- bzw. Bodenteil. Indem nun ein erfindungsgemässer Filter so an den Lochbereich angelegt wird, dass dessen Dichtung von der Stirnseite eines geschlossenen Mantelbereiches des einen gegen eine geschlossene, um den Lochbereich verlaufendeTeilfläche des anderen Teiles gepresst wird, kann ein steriler Abschluss gewährleistet werden.

Zum Herstellen des Filters mit Dichtung wird auf das von einer Vorrichtung gehaltene Flachmaterial mit einer Aufschäumvorrichtung eine linienförmig geschlossene Dichtung aufgebracht, die nach dem Aufbringen polymerisiert und dabei eine Verbindung mit dem Flachmaterial ausbildet. Mit grösserem Aufwand wäre es auch möglich einen Dichtungsring auf das Flachmaterial aufzukleben. Das Flachmaterial wird gegebenenfalls vor dem Auftragen der Dichtung auf die gewünschte Grösse zugeschnitten. Vorzugsweise werden aber auf einem Flachmaterialband Dichtungen aufgebracht. Das Abtrennen der einzelnen Filter erfolgt vorzugsweise erst später. Nach dem Aufbringen der Dichtung wird entsprechend dem aufgetragenen Dichtungsmaterial vorzugsweise mindestens eine Temperaturbehandlung durchgeführt. Bei der Verwendung eines Silikonschaumes wird etwa eine erste Temperaturbehandlung während mindestens 3, insbesondere während 5, Minuten bei mindestens 50°C, insbesondere aber bei im wesentlichen 60°C und gegebenenfalls eine zweite Temperaturbehandlung während mindestens 10, insbesondere während 20-30, Minuten bei mindestens 120°C, insbesondere aber bei im wesentlichen 140°C durchgeführt. Für medizinische Anwendungen werden vorzugsweise Silikonschäume und insbesondere solche, die lebensmittelecht und/oder vom Gesundheitsamt zugelassen sind, verwendet.

Aufgeschäumte Silikondichtungen haben die für die Sterilisation benötigte Temperatur-, Feuchtigkeits- und Druckwiderstandsfähigkeit. Das heisst, sie nehmen nach der Sterilisation genügend schnell wieder die ursprüngliche Form an. Sie haben auch nach dem mehrmaligen Durchlaufen von Sterilisationsvorgängen noch die gewünschte Elastizität und Form. Selbst wenn sie gegebenenfalls während der Sterilisation aufgrund einer druck- oder temperaturbedingten Verformung keinen dichten Abschluss gewähren, so ist der dichte Abschluss aber zumindest für die anschliessende Lagerung wieder gewährleistet. Die geschäumten Dichtungen haben eine sehr grosse Verformbarkeit und sind daher auch für Abdichtungen zwischen Pressflächen mit hohen Toleranzen bezüglich des planen Zusammenführens besonders geeignet.

Die Zeichnungen erläutern die Erfindung anhand von Ausführungsbeispielen auf die sie aber nicht eingeschränkt ist. Dabei zeigt
- Fig. 1a: eine Draufsicht auf einen Filter mit Dichtung,
- Fig. 1b: einen Schnitt A-A gemäss Fig. 1a durch einen Randausschnitt des Filters,
- Fig. 2: eine Draufsicht auf einen Trayteil (Boden oder Deckel) mit einem Lochbereich und einer darumherum führenden Nut,
- Fig. 3: einen vertikalen Schnitt durch einen Waschkorb,
- Fig. 4: eine Draufsicht auf einen Waschkorb,
- Fig. 5: einen vertikalen Schnitt durch einen Waschkorb mit unten und oben aufgesetztem Abschlussteil mit Filter,
- Fig. 6: eine Ansicht eines Abschlussteiles von oben,
- Fig. 7: eine Ansicht eines auf einem Waschkorbmantel sitzenden Abschlussteiles von unten,
- Fig. 8: eine Seitenansicht eines Trays mit einem Mantelteil und zwei Abschlussteilen,
- Fig. 9: eine Seitenansicht (links) und einen vertikalen Schnitt (rechts) eines Abschlussteiles ohne Verschlussriegel,
- Fig. 10: eine Seitenansicht eines Verschlussriegels eines Abschlussteils, und
- Fig. 11: eine Draufsicht auf einen Verschlussriegel eines Abschlussteils

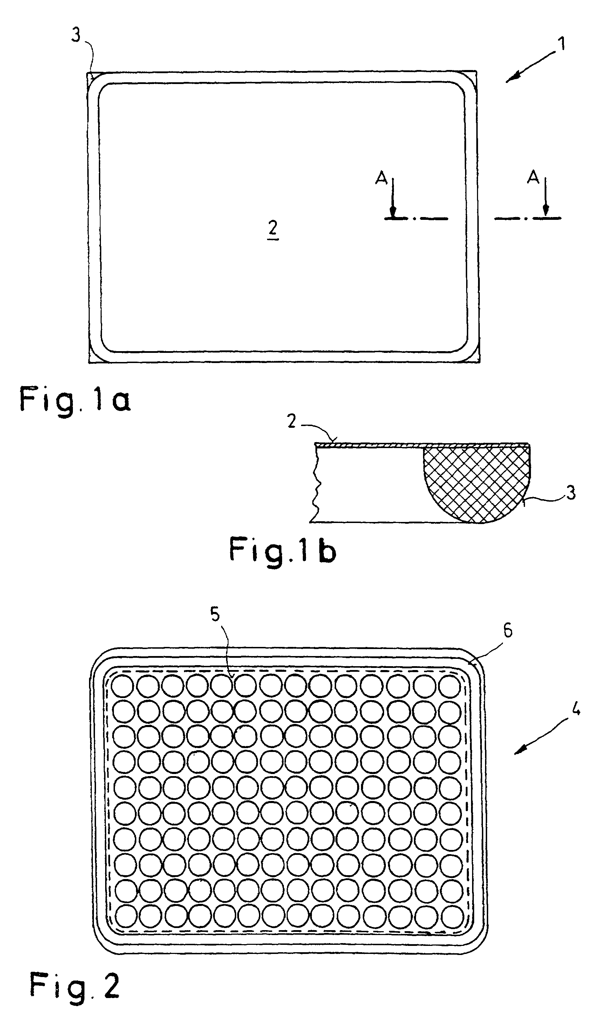

Fig. 1 zeigt einen Filter 1, der einen Filterbereich aus flexiblem Flachmaterial 2 und mindestens eine entlang einer geschlossenen Linie verlaufende Dichtung 3 umfasst. Die Dichtung 3 ist fest mit dem Flachmaterial verbunden. So könnte beispielsweise etwa eine ringförmige Dichtung aufgeleimt werden. Vorzugsweise wird aber die Verbindung dadurch erzielt, dass die Dichtung direkt auf das Flachmaterial 2 aufgeschäumt wird. Dabei verbindet sich das viskose Schaummaterial mit dem Flachmaterial, bzw. mit dessen Fasern. Beim anschliessenden Polymerisieren bzw. Abbinden wird die Verbindung zum Flachmaterial und natürlich die Form der Dichtung 3 fixiert. Die Dichtung 3 verläuft vorzugsweise entlang des Flachmaterialrandes, wobei in Eckbereichen ein abgerundeter Verlauf gewählt wird.

Gemäss Fig. 1b ist die Dichtung 3 vorzugsweise wulstförmig. Es wären aber auch flache, bandartige Dichtungen möglich.

Fig. 2 zeigt einen Trayteil 4 mit einem Lochbereich 5 und einer darumherum führenden Nut 6. Um den Lochbereich 5 mit einem Filter 1 abzuschliessen, wird die Dichtung 3 des Filters 1 in die Nut 6 eingepresst. Damit der so eingesetzte Filter 1 in jeder Lage des Trayteils 4 hält, ist die Nut 6 entsprechend der Dichtung 3 ausgebildet. Das Trayteil 4 mit dem eingesetzten Filter 1 wird mit einem weiteren Trayteil so zusammengestellt, dass die Dichtung 3 zwischen zwei zusammenwirkenden Pressflächen liegt. Die eine Pressfläche wird von der Nut und die andere von einer Mantelstirnseite gebildet.

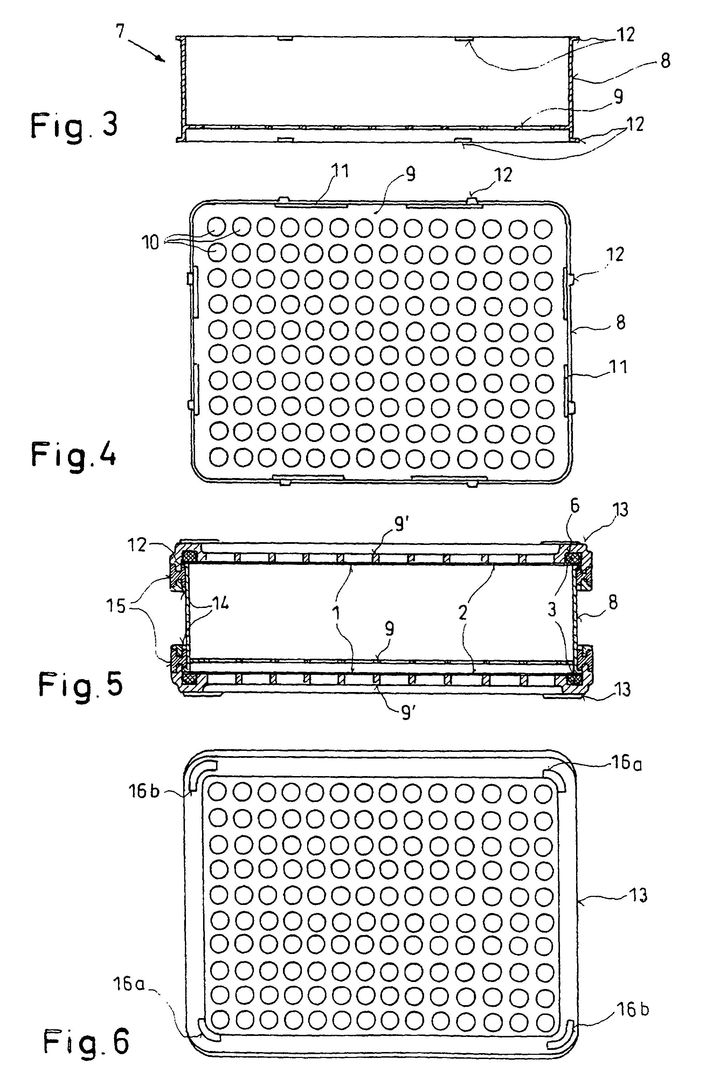

Fig. 3 zeigt einen Waschkorb 7 mit einer geschlossenen Mantelwand 8, die im Bereich der unteren Stirnseite mit einem Lochblech 9 abgeschlossen ist. In der Draufsicht gemäss Fig. 4 sind die regelmässige Lochanordnung 10 und die schlitzförmigen Durchtrittsöffnungen 11 zu erkennen. Nebst den dargestellten kreisrunden Löchern sind auch abgeflachte oder eckige Löcher möglich. Es versteht sich von selbst, dass die Löcher in den Abschlusselementen und auch im Boden des Waschkorbes vorzugsweise analog ausgebildet werden könen. Am oberen und unteren Mantelrand sind Stege 12 ausgebildet, die von der Mantelwand 8 des Waschkorbes 7 nach aussen vorstehen. Sie dienen einerseits zur Zentrierung von auf die Mantelstimseiten aufsetzbaren Abschlussteilen. Andererseits dienen die Stege 12 zweier gegenüberliegender Seiten als Eingriffselemente einer Klemmvorrichtung zum Festsetzen der Abschlussteile an den Stirnseiten der Mantelwand 8.

Fig. 5 zeigt einen Waschkorb 7, an dem oben und unten je ein Abschlussteil 13 mit einem Lochbereich 9' als Deckel bzw. Boden festgesetzt ist. Am Lochbereich 9' ist je ein Filter 1 mit einer Filterfläche 2 und einer in eine Nut 6 eingelegten Dichtung 3 vorgesehen. Die Klemmvorrichtung umfasst nebst den Stegen 12 am Waschkorb 7, an zwei einander gegenüberliegenden Seiten jedes Abschlussteiles 13 Verschiebeteile 15 mit rampenartigen Klemmflächen 14. Diese sind so beweglich gelagert, dass die Klemmflächen 14 zum Erzielen einer gewünschten Kontaktlage jedes Abschlussteils 13 an der Mantelwand 8 entlang der Stege 12 bewegbar sind. Bei der Verschliessbewegung dieser Verschiebeteile 15 wird das Abschlussteil 13 von den Klemmflächen 14 im Zusammenwirken mit den Stegen 12 gegen die Mantelwand 8 bewegt, so dass die Dichtung 3 zwischen der Mantelwand-Stirnseite und dem Nutgrund der Nut 6 festgepresst wird.

Gemäss Fig. 6 haben die Abschlussteile 13 in den Eckbereichen der Aussenflächen Stapelhilfen 16a und 16b, von denen diejenigen der einen Diagonalen innerhalb und diejenigen der anderen Diagonalen ausserhalb einer Kontaktlinie angeordnet sind. Dadurch können zwei Abschlussteile 13 mit ihren Aussenflächen im wesentlichen verrutschungsfrei aneinander angelegt werden. Entsprechend sind Trays gemäss Fig. 5 aufeinander stapelbar. Die Stapelhilfen 16a, 16b sind so ausgebildet, dass zwischen den gestapelten Trays, bzw. zwischen den einander zugewandten Abschlussflächen der Abschlussteile 13 ein schichtförmiger Freiraum offen bleibt. Dieser Freiraum ermöglicht eine Luft- und Dampfzirkulation zwischen gestapelten Trays. Entsprechend können solche Trays auch in Sterilisatoren ohne Einschubleisten gestapelt und sterilisiert werden. Um die Lagerung der Abschlussteile 13 zu erleichtern, sind die Abschlussteile 13 so ausgebildet, dass sie auch in gleicher Ausrichtung aufeinander stapelbar sind.

Die Ausführungsform mit dem Waschkorb 7 und den zwei gleichen Abschlussteilen 13 hat den Vorteil, dass mit lediglich zwei verschiedenen Trayteilen und zwei Filtern 1 eine Lösung bereitgestellt wird, die den Einsatz in einer Reinigungsvorrichtung und in einer Sterilisationsvorrichtung, sowie eine Sterillagerung ermöglicht. Der Waschkorb 7 wird vorzugsweise aus Stahl und die Abschlussteile 13 aus Kunststoff hergestellt.

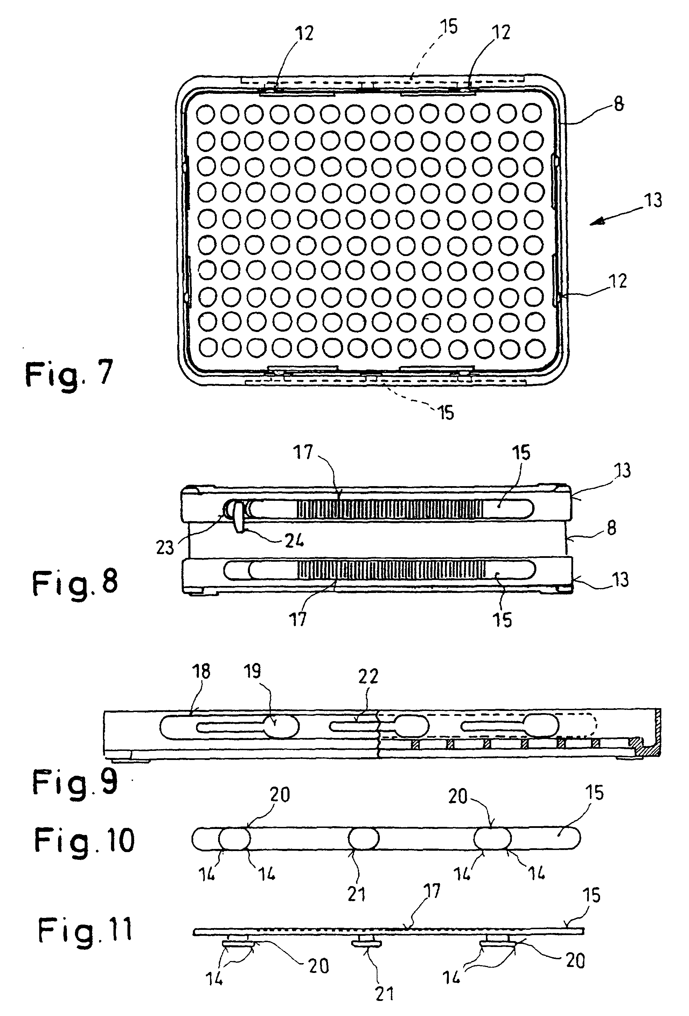

Fig. 7 weist schematisch darauf hin, dass Schieber 15 vorzugsweise in zwei einander gegenüberliegenden Seitenflächen des Abschlussteiles 13 eingesetzt sind. Die Stege 12 aller Seiten stehen zur Zentrierung des Abschlussteiles 13 gegen die nach innen gerichtete Mantelfläche des Abschlussteiles 13 vor. Die Stege 12 dienen auch als Verbreiterungen der Auflagefläche für die Dichtung 3 und verhindern so ein seitliches Verklemmen der Dichtung 3 zwischen der Mantelwand 8 und der nach innen gerichteten Mantelfläche des Abschlussteiles 13.

Fig. 8 zeigt, dass zur Betätigung der Schieber 15 Griffrippen 17 von aussen zugänglich sind. Die Schieber 15 sind gemäss Fig. 9 in Vertiefungen 18 und Durchführungen 19 verschiebbar angeordnet. Gemäss Fig. 10 und 11 sind die Klemmflächen 14 an durch die Durchführungen 19 nach innen vorstehenden Klemmteilen 20 ausgebildet. Im mittleren Bereich des Schiebers 15 ist gegebenenfalls ein, die Schiebebewegung begrenzendes Führungsteil 21 und im Abschlussteil ein entsprechender Führungsbereich 22 vorgesehen.

Um zu verhindern, dass ein Tray mit sterilisierten Instrumenten geöffnet und wieder verschlossen werden kann, ohne dass dies ersichtlich wäre, wird eine Sicherung vorgesehen. Diese Sicherung kann im Sinne einer Plombierung oder Versiegelung ausgebildet werden. Vorzugsweise wird vor der Sterilisierung ein entreissbares Sicherungsteil 23 in die Vertiefung 18 neben den Schieber 15 eingesetzt. Dieses Sicherungsteil 23 setzt den Schieber 15 in der klemmenden Position fest. Bevor ein Abschlussteil 13 vom Waschkorb 7 abgenommen werden kann, muss das Sicherungsteil 23 entfernt werden. Dazu ist am Sicherungsteil 23 eine Lasche 24 ausgebildet. Das Sicherungsteil 23 und seine Aufnahme im Abschlussteil 13 sind vorzugsweise so ausgebildet, dass beim Entnehmen eine Veränderung am Sicherungsteil 23 eintritt, die ein Wiedereinsetzen verhindert. Dadurch kann gewährleistet werden, dass ein Öffnen erkennbar bleibt.

## Patentansprüche

1. Tray mit einem Waschkorb (7), der eine geschlossene Mantelwand (8) und im unteren Bereich einen Waschteilboden (9) mit Durchtrittsöffnungen umfasst, und mit zwei Aufnahmebereichen zum Aufnehmen von je einem Filter (1) aus Flachmaterial, wobei die Filter (1) je bei einer Stirnseite der Mantelwand (8) anzuordnen sind, **dadurch gekennzeichnet, dass** zwei Abschlussteile (13) den Waschkorb oben und unten abschliessen und die Stirnseiten der Mantelwand (8) mit den Abschlussteilen (13) zusammenwirkende Pressflächen bilden, die sich je entlang einer geschlossenen Dichtlinie erstrecken und zwischen denen je entlang der gesamten Dichtlinie eine Dichtung und daran anliegend das Filter-Flachmaterial (2) festklemmbar ist, wobei die Abschlussteile (13) je einen von der Dichtlinie umgebenen Lochbereich (5) aufweisen, der somit von dem Filter-Flachmaterial (2) abgedeckt wird.

2. Tray nach Anspruch 1, **dadurch gekennzeichnet, dass** in den Abschlussteilen (13), oder gegebenenfalls im Waschkorb (7), im Bereich der Dichtlinie eine Nut (6) ausgebildet ist, in der die Dichtung (3), insbesondere eine mit dem Filterflachmaterial (2) verbundene wulstförmige Dichtung, festsetzbar ist.

3. Tray nach Anspruch 1 oder 2, **dadurch gekennzeichnet, dass** eine Klemmvorrichtung vorgesehen ist, welche die Abschlussteile (13) gegen eine entsprechende Stirnseite der Mantelwand (8) pressbar macht.

4. Tray nach Anspruch 3, **dadurch gekennzeichnet, dass** die Klemmvorrichtung am Waschkorb (7) und an jedem Abschlussteil (13) zusammenwirkende Eingriffselemente (12, 20) umfasst, wobei vorzugsweise von der Mantelwand (8) des Waschkorbes (7) Stege (12) nach aussen vorstehen und an jedem Abschlussteil (13) Verschiebeteile (15) mit rampenartigen Klemmflächen (14) so beweglich gelagert sind, dass die Klemmflächen (14) zum Erzielen einer gewünschten Kontaktlage des Abschlussteils (13) an der Mantelwand (8), bzw. einer elastischen Verformung der Filterdichtung (3), entlang der Stege (12) bewegbar sind.

5. Tray nach einem der Ansprüche 1 bis 4, **dadurch gekennzeichnet, dass** zumindest ein Sicherungsteil (23) einsetzbar ist, das ein Öffnen des Trays nur nach Entfernung dieses Sicherungsteiles (23) ermöglicht, wobei beim Entfernen eine Veränderung am Sicherungsteil (23) eintritt, die ein Wiedereinsetzten verhindert.

## Claims

1. Tray comprising a wash basket (7) including a closed peripheral wall (8) and in the bottom area a wash part bottom (9) having through openings, and two mounting areas each for taking a filter (1) of flexible flat material (2), wherein the filters (1) are located each at a front surface of the peripheral wall (8), **characterised in that** two closure members (13) are covering the wash basket (7) at the top and at the bottom and that the front surfaces of the peripheral wall (8) together with the closure members (13) are bilding cooperating damping surfaces, extending along dosed sealing lines and enabling damping a seal (3) and a filtering flat material (2) in contact with the seal (3), wherein the closure members (13) include each an area with through openings *covered by the filtering flat material (2) and* surrounded by the sealing line.

2. Tray according to claim 1, **characterised in that** a groove (6) is formed at the closure members (13), or optionally at the wash basket (7), within the region of the sealing line wherein the seal (3), particularly a doughnut-shaped seal bond to the filtering flat material (2), is to be fixed in said groove (6).

3. Tray according to claim 1 or 2, **characterised in that** clamping means being provided which enables pressing the closure members against the corresponding front surface of the peripheral wall (8).

4. Tray according to claim 3, **characterised in that** the damping means of the wash basket (7) and of each dosure member (13) comprises co-operating engagement elements (12, 20), straps (12) projecting preferably from the peripheral wall (8) of the wash basket (7) to the exterior and slider members (15) which include ramp-like damping surfaces (14) being movably supported in such a way that the damping surfaces (14) are movable along the straps (12) for achieving a desired contact position of the closure member (13) to the peripheral wall (8) and an elastic deformation of the filter seal (3).

5. Tray according to any of claims 1 to 4, **characterised in that** at least one safety piece (23) is insertable which enables opening of the tray only after removal of the safety piece (23), wherein safety piece (23) is changed by removing which prevents inserting it anew.

## Revendications

1. Plateau avec un panier de lavage (7) comportant une paroi d'enveloppe fermée (8), et dans la zone inférieure un fond partiel de lavage (9) avec des orifices de passage, et deux zones de réception destinées chacune à la réception d'un filtre (1) en matériau plat, les filtres (1) étant à disposer à proximité d'une face frontale de la paroi d'enveloppe (8), **caractérisé en ce que** deux éléments de fermeture (13) ferment le panier de lavage en haut et en bas, et **en ce que** les faces frontales de la paroi d'enveloppe (8) forment avec les éléments de fermeture (13) des surfaces de pression agissant conjointement, qui s'étendent le long d'une ligne d'étanchéité fermée, et entre lesquelles un joint et du matériau filtrant plat (2) y adhérant peuvent être enserrés le long de l'ensemble de la ligne d'étanchéité, les éléments de fermeture (13) comportant chacun une zone perforée (5) entourée par la ligne d'étanchéité, laquelle est ainsi recouverte par le matériau filtrant plat (2).

2. Plateau selon la revendication 1, **caractérisé en ce que,** dans la zone de la ligne d'étanchéité, une rainure (6) est pratiquée dans les éléments de fermeture (13), ou le cas échéant dans le panier de lavage (7), dans laquelle le joint (3) peut être bloqué, notamment un joint en forme de bourrelet relié au matériau filtrant plat (2).

3. Plateau selon la revendication 1 ou 2, **caractérisé en ce qu'**il est prévu un dispositif de serrage avec lequel les éléments de fermeture (13) peuvent être serrés contre une face frontale correspondante de la paroi d'enveloppe (8).

4. Plateau selon la revendication 3, **caractérisé en ce que**, sur le panier de lavage (7) et sur chaque élément de fermeture (13), le dispositif de serrage comporte des éléments d'engagement (12, 20) agissant conjointement, des barrettes (12) dépassant de préférence de la paroi d'enveloppe (8) du panier de lavage (7) vers l'extérieur, et des éléments mobiles (15) comportant des surfaces de serrage (14) de type rampe étant positionnés de façon mobile sur chaque élément de fermeture (13) de telle sorte que, pour obtenir une position de contact souhaitée de l'élément de fermeture (13) sur la paroi d'enveloppe (8) ou une déformation élastique du joint (3) du filtre, les surfaces de serrage (14) puissent être déplacées le long des barrettes (12).

5. Plateau selon l'une quelconque des revendications 1 à 4, **caractérisé en ce qu'**un élément de sécurité (23) au moins peut être inséré, qui ne permet une ouverture du plateau qu'après le retrait de cet élément de sécurité (23), une modification empêchant une réinsertion intervenant au niveau de l'élément de sécurité (23) lors du retrait.
